# FASCICULE DE BREVET EUROPEEN

(11) **EP 1 508 325 B1**
(45) Date de publication et mention de la délivrance du brevet: **11.03.2009**
(21) Numéro de dépôt: 03018982.3
(22) Date de dépôt: 21.08.2003
(51) Int. Cl.: A61Q 19/08, A61K 8/97

(54) **Concentré naturel de Lycopène et procédé d'obtention**
Natürliche Lycopenkonzentrate und Verfahren zur Herstellung
Natural lycopene concentrate and process for producing the same

(43) Date de publication de la demande: 23.02.2005
(73) Titulaire: Nestec S.A., 1800 Vevey (CH)
(72) Inventeur: Bortlik, Karlheinz, 1073 Savigny (CH); Duruz, Eliane, 1066 Epalinges (CH); Kolodziejczyk, Eric, 1800 Vevey (CH); Fernandez-Graf, Marie-Rose, 1009 Pully (CH)
(74) Mandataire: Chautard, Cécile

(56) Documents cités:
- EP-A- 1 051 918
- WO-A-02/40003
- WO-A-03/041678
- DATABASE WPI Section Ch, Week 197913 Derwent Publications Ltd., London, GB; Class D13, AN 1979-25093B XP002269911 & JP 54 024940 A (KAGOME KK), 24 février 1979 (1979-02-24)
- "ABOUT LYC-O-MATO" ABOUT LYC-O-MATO, 5 mars 2003 (2003-03-05), pages 1-5, XP002233574 Extrait de l'Internet: <URL:http://www.lycomato.com/> [extrait le 2003-03-05]

## Description

La présente invention concerne un concentré naturel de lycopène ainsi que son procédé d'obtention et son utilisation.

Le lycopène est un pigment naturel contenu en grande quantité dans les tomates mais il est également présent dans le melon, la goyave, la pastèque ou le pamplemousse. Il est connu pour ses propriétés bioactives et en particulier pour son rôle d'antioxydant.
Il existe sur le marché des préparations contenant du lycopène. Ces préparations sont généralement sous forme d'oléorésines et le lycopène qu'elles contiennent est d'une biodisponibilité relativement limitée. De plus, le lycopène est extrait à l'aide de solvants organiques, lesdits solvants sont donc susceptibles d'être retrouvés sous forme de traces dans le produit fini.

En effet, EP 1103579 décrit un mélange de lycopène avec une solution de protéines sériques afin d'augmenter la biodisponibilité de celui-ci. Le lycopène décrit est extrait au moyen d'un solvant.

WO03/041678 décrit un lycopène avec une biodisponibilité accrue grâce à l'ajout de divers additifs tels que des huiles ou des surfactants. Le lycopène décrit est traité par des solvants.

Par ailleurs, JP 54024940 décrit un procédé de concentration de lycopène à partir de résidus de tomates tels que les graines ou la peau. Ce procédé met en jeu les enzymes endogènes de ces résidus afin de dégrader les tissus biologiques pour faciliter l'extraction de lycopène. Cette activité enzymatique est induite par une incubation de 5 heures à des températures comprises entre 45 et 60 °C. Ensuite le lycopène est extrait après une séparation des peaux et graines par filtration suivie d'une floculation fractionnée des différents insolubles indésirables.

La présente invention a pour but de proposer un produit "naturel" avec une biodisponibilité accrue, c'est à dire que le produit n'a subit que des traitements technologiques ne modifiant pas ses caractéristiques natives.

De plus, le procédé d'extraction selon l'invention est simple, rapide et économique et à aucun moment soumis à l'état de viabilité des endoenzymes de la matière première.
La présente invention concerne un concentré naturel de lycopène, ledit est hydrosoluble à température ambiante tandis que le lycopène était jusqu'alors liposoluble. Cette hydrosolubilité est obtenue sans ajout de surfactants.

Le concentré peut être obtenu à partir de tout végétal contenant du lycopène à savoir: la tomate, le melon, la pastèque, la goyave, le pamplemousse, l'abricot, le cynorrhodon.

Dans le cas de la tomate, la matière première utilisée peut être une pâte de tomate.

Par pâte de tomate dans la présente description, on entend un extrait concentré de tomates incluant des protéines, des carbohydrates, des polysaccharides, des composés liposolubles comme les caroténoïdes et entre autre le lycopène, ainsi que des acides organiques.
Dans la composition selon l'invention, la pâte de tomate est choisie selon sa concentration initiale en lycopène. En effet, la teneur du concentré en lycopène dépend de la teneur initiale de la matière première en ce même composé.

Le concentré selon l'invention contient au moins 1 mg de lycopène par g dudit concentré. Le concentré contient de préférence entre 1 mg et 40 mg de lycopène par g de concentré et plus préférentiellement entre 10 et 30 mg de lycopène par g de concentré.
Le concentré contient en outre jusqu'à 30 % de protéines, jusqu'à 30 % de polysaccharides, jusqu'à 10% d'acides organiques, au moins 30 % de composés lipidiques et donc entre 0.0001 et 2% de lycopène et plus préférentiellement entre 0.001 et 1% de lycopène.
Les pourcentages sont donnés par rapport au poids sec.

La composition selon l'invention peut se présenter sous forme pulvérulente, liquide ou gélifiée.

Dans le cas du concentré sous forme liquide, ledit concentré contient au moins 35 % d'eau. Ledit concentré contient plus préférentiellement de 60 à 95 % d'eau.

Comme mentionné auparavant, les deux caractéristiques importantes de cette invention sont d'avoir un concentré de Lycopène ayant une biodisponibilité satisfaisante, et étant facile d'utilisation puisque dans le cas de la poudre, le concentré est une poudre de lycopène hydrosoluble à température ambiante.
Cela sans utiliser de solvant au cours du procédé afin de conserver la naturalité de produit pour mettre à disposition du consommateur un concentré de bioactivité importante.

La meilleure biodisponibilité du lycopène concentré selon l'invention est expliquée par le fait que les cristaux obtenus selon le procédé revendiqué sont d'une taille 5 à 10 fois inférieure à celle des formes cristallines de l'oléorésine. Sans oublier que la matière première utilisée, en l'occurrence la pâte de tomate, est la source dans laquelle le lycopène est le plus biodisponible du fait des différents traitements technologiques qu'elle a subit durant son élaboration.

Par oléorésine on entend un extrait lipidique de plante incluant des caroténoïdes, comme le lycopène, des triglycérides, des phospholipides, du tocophérol et d'autres composés plus mineurs.

La poudre ou le gel ou la solution, selon l'invention, peuvent en outre contenir de la vitamine E et/ou de la vitamine C qui pourraient être aj outées.

La présente invention concerne également le procédé de fabrication du concentré décrit ci-avant dans lequel:
- on alcalinise une structure végétale contenant un composé lipophile,
- on chauffe la solution alcalinisée jusqu'à ébullition que l'on maintient pendant 1 à 60 minutes,
- on isole les fibres et divers composés insolubles par séparation solide-liquide et préférentiellement par filtration à chaud,
- on acidifie la solution obtenue,
- on isole les carbohydrates et autres composés solubles par séparation solide-liquide et préférentiellement par centrifugation,
Par structure végétale on entend une pâte dudit végétal obtenue par réduction de sa teneur en eau.

Selon un premier mode de réalisation du procédé on obtient un liquide. Ce liquide a un comportement rhéofluidifiant et sa viscosité est de 90 à 120 mPa.s sous un taux de cisaillement de 160 s⁻¹.Cette viscosité a été mesurée à une température constante de 20.5°C avec un appareil RheoStress 150 de géométrie Couette avec un taux de cisaillement croissant de 1 à 1000 s⁻¹.

On peut traiter ledit liquide par ajout de calcium pour obtenir un gel car cet ajout va avoir pour effet une gélification des polysaccharides. Dans un second mode de réalisation du procédé, on peut sécher le concentrat par atomisation ou lyophilisation pour obtenir une poudre.
La composition selon l'invention peut être utilisée directement sous ces différentes formes ou en mélange.
On peut utiliser une pâte de tomate contenant au minimum 0.1mg de lycopène par g de produit à un pH compris entre 3.5 et 5 et plus préférentiellement compris entre 4 et 4.5. On alcalinise cette pâte avec de l'eau déminéralisée et une base du type NaOH jusqu'à obtenir un pH entre 6 et 9.
On mélange cette préparation en chauffant jusqu'à la température d'ébullition. Cette ébullition est maintenue durant 1 à 60 minutes et plus préférentiellement durant 2 à 30 minutes.
Ensuite on fait une séparation solide-liquide pour isoler les fibres et autres composés insolubles et on fait plus préférentiellement une filtration à chaud.

Le filtrat récupéré est une solution contenant du lycopène dispersé. On acidifie par un acide type acide citrique jusqu'à un pH compris entre 3.5 et 5 et plus préférentiellement entre 4 et 4.5.
On mélange cette préparation puis on fait une séparation solide-liquide afin de récupérer les carbohydrates et autres composés solubles et on fait plus préférentiellement une centrifugation.

La solution obtenue est un concentré de lycopène selon l'invention. On peut ramener le pH à la neutralité avec de l'eau déminéralisée et une base du type NaOH, en mélangeant simultanément.
Le lycopène est maintenant hydrosoluble car il a été complexé par des protéines du milieu ainsi que par des polysaccharides.

Selon la forme physique souhaitée, le concentré de lycopène est soit utilisé en l'état, en l'occurrence sous forme liquide.
Soit sous forme gélifiée, on va alors ajouter du calcium au complexe afin de provoquer la gélification des polysaccharides.
Enfin, pour obtenir une poudre on sèche le concentrat par atomisation ou tous autres moyens connus par l'homme du métier.
La présente invention concerne aussi l'utilisation de la poudre décrite précédemment dans une composition cosmétique afin de ralentir le vieillissement de la peau et/ou les dégradations cutanées occasionnées par l'exposition aux UV, ladite composition contenant au moins 10-¹⁰% de lycopène.
Cette composition utilisable par voie topique peut en outre contenir une graisse ou une huile acceptable en cosmétique. L'ajout d'autres ingrédients cosmétiques actifs est également possible. La composition peut aussi contenir un agent structurant, un tensioactif, des excipients, des colorants, des parfums, des abrasifs ou encore des opacifiants.

La composition selon l'invention contient entre 10⁻¹⁰ et 10% de lycopène, plus préférentiellement la composition cosmétique contient entre 10⁻⁸ et 5% de lycopène.

La présente invention concerne également l'utilisation du concentré de lycopène dans des compositions ingérables par voie orale afin d'utiliser la haute biodisponibilité du lycopène sous cette forme. Le but est d'induire une photoprotection et de ralentir le vieillissement de la peau. Les milieux susceptibles de contenir ce concentré peuvent être: des boissons, du chocolat, des glaces ou crèmes glacées, des préparations céréalières, du café soluble ou des plats cuisinés.
Dans ce cas, la poudre est dissoute dans les préparations concernées de manière à avoir une prise journalière comprise entre 0.001 et 50 mg de lycopène. La prise journalière préférée sera entre 2 mg et 10 mg.

La présente invention peut également se présenter sous forme de pilules, de gélules ou de tablettes dosées de 0.001 à 100 % du dit concentrat. Ces produits peuvent alors être ingérés directement avec de l'eau ou par tout autre moyen connu.
Le concentré peut enfin être envisagée comme produit préventif des affections de la peau liées à une exposition excessive aux U.V. Dans ce cas, l'utilisation peut se faire tant par voie orale que topique.
La suite de la description est maintenant faite en référence aux exemples:

### Exemple 1 : Préparation du concentré sous forme de poudre:

On mélange 50 kg de purée de tomate à pH 4.3 avec 100 kg d'eau déminéralisée dans un batch. Ce mélange est effectué à une température de 25 °C. Le pH est amené à 7 avec du NaOH.
La solution est chauffée jusqu'à ébullition et cette ébullition est maintenue durant 5 minutes.
On laisse reposer la solution 15 minutes à température ambiante, ensuite on filtre à chaud dans un décanteur type Westfalia.
Le filtrat recueillit est refroidit dans de l'eau à une température de 15°C.
On mélange cette solution en acidifiant avec de l'acide citrique jusqu'à un pH de 4.3.
La solution est ensuite centrifugée dans un centrifugeur type Padberg à 14 000 tours par minutes.
On récupère le surnageant et on ajuste son pH à 7.00 avec du NaOH.
Enfin, on atomise la solution obtenue dans un atomiseur type NIRO avec une température d'entrée de 110 à 130°C et une température de sortie comprise entre 70 et 80 °C. La vitesse de rotation de la buse d'atomisation est comprise entre 24 000 et 30 000 tours par minute selon la finesse de poudre souhaitée.
On récupère alors le concentré selon l'invention.

### Exemple 2 : Composition cosmétique:

On prépare un lait pour le visage contenant 7 % d'huile de vaseline, 2 % de poudre selon l'exemple 1, 3 % de monostéarate de glycéryle, stéarate de polyéthylène-glycol, 0.4 % de polymère carboxyvinylique, 0.7 % d'alcool stéarylique, 3 % de protéines de soja, 0.4 % de NaOH, un conservateur et le complément à 100 est de l'eau.

### Exemple3: Composition cosmétique:

On prépare un gel pour le visage contenant 10 % de glycérine, 2 % de poudre selon l'exemple 1, 1 % de cocoamphodiacétate de disodium, un conservateur et le complément à 100 est de l'eau.

## Revendications

1. Concentré naturel de lycopène extrait de purée de tomate, de melon, de pastèque, de goyave, de pamplemousse, d'abricot et/ou de cynorrhodon,
**caractérisé en ce que**
A - il est hydrosoluble à température ambiante,
B - il contient au moins 1 mg de lycopène par g dudit concentré, jusqu'à 30% de protéines, jusqu'à 30% de polysaccharides, jusqu'à 10% d'acides organiques, et au moins 30% de composés lipidiques.

2. Concentré selon la revendication 1, **caractérisé en ce qu'**il se présente sous forme pulvérulente, liquide ou gélifiée.

3. Concentré selon la revendication 1, **caractérisé en ce qu'**il peut contenir en outre de la vitamine E et/ou de la vitamine C.

4. Concentré selon la revendication 1, **caractérisé en ce qu'**il contient entre 1 mg et 40 mg de lycopène par g de concentré.

5. Concentré selon la revendication 4, **caractérisé en ce qu'**il contient préférentiellement entre 10 et 30 mg de lycopène par g de concentré.

6. Procédé de préparation du concentré selon l'une des revendications 1 à 5, dans lequel :
- on alcalinise en solution aqueuse une structure végétale contenant un composé lipophile choisi parmi la purée de tomate, le melon, la pastèque, la goyave, le pamplemousse, l'abricot et le cynorrhodon,
- on chauffe la solution alcalinisée jusqu'à ébullition que l'on maintient pendant 1 à 60 minutes,
- on isole les fibres et divers composés insolubles par séparation solide-liquide,
- on acidifie en solution aqueuse le complexe obtenu,
- on isole les carbohydrates et autres composés solubles par séparation solide-liquide.

7. Procédé selon la revendication 6, **caractérisé en ce que** l'on ajoute du calcium au complexe pour obtenir un gel.

8. Procédé selon la revendication 6, **caractérisé en ce que** l'on sèche l'émulsion par atomisation ou lyophilisation pour obtenir une poudre.

9. Composition cosmétique topique contenant du concentré selon l'une des revendications 1 à 5 pour le ralentissement du vieillissement de la peau et/ou la lutte contre les dégradations cutanées pouvant être occasionnées par une exposition aux U.V., ladite composition contenant au moins 10⁻¹⁰% de lycopène.

10. Composition ingérable par voie orale contenant le concentré selon l'une des revendications 1 à 5 pour optimiser l'absorption du lycopène afin d'induire une photoprotection et ainsi ralentir le vieillissement de la peau, par exemple dans des boissons, du chocolat, des glaces ou crèmes glacées, des préparations céréalières, du café soluble ou encore des plats cuisinés.

11. Supplément alimentaire contenant le concentré selon l'une des revendications 1 à 5 sous forme de pilules, de gélules ou de tablettes dosées de 0.0001 à 100% dudit concentré.

## Claims

1. Natural lycopene concentrate extracted from pureed tomato, melon, water melon, guava, grapefruit, apricot and/or rose hip, **characterized in that**
A - it is water-soluble at room temperature, and
B - it contains at least 1 mg of lycopene per g of said concentrate, up to 30% of proteins, up to 30% of polysaccharides, up to 10% of organic acids and at least 30% of lipidic compounds.

2. Concentrate according to Claim 1, **characterized in that** it takes the form of a powder, a liquid or a gel.

3. Concentrate according to Claim 1, **characterized in that** it can also contain vitamin E and/or vitamin C.

4. Concentrate according to Claim 1, **characterized in that** it contains between 1 mg and 40 mg of lycopene per g of concentrate.

5. Concentrate according to Claim 4, **characterized in that** it preferably contains between 10 and 30 mg of lycopene per g of concentrate.

6. Process for the preparation of the concentrate according to one of Claims 1 to 5 wherein:
- a plant structure containing a lipophilic compound selected from pureed tomato, melon, water melon, guava, grapefruit, apricot and rose hip is alkalized in aqueous solution,
- the alkalized solution is heated to the boil and kept at the boil for 1 to 60 minutes,
- the fibres and various insoluble compounds are isolated by solid-liquid separation,
- the complex obtained is acidified in aqueous solution, and
- the carbohydrates and other soluble compounds are isolated by solid-liquid separation.

7. Process according to Claim 6, **characterized in that** calcium is added to the complex to give a gel.

8. Process according to Claim 6, **characterized in that** the emulsion is dried by atomization or lyophilization to give a powder.

9. Topical cosmetic composition containing concentrate according to one of Claims 1 to 5 for slowing down the skin ageing and/or combating the skin degradations that can be caused by exposure to UV, said composition containing at least 10⁻¹⁰% of lycopene.

10. Orally ingestible composition containing the concentrate according to one of Claims 1 to 5 for optimizing the absorption of lycopene so as to induce photoprotection and thereby slow down skin ageing, e.g. in drinks, chocolate, ices or ice-creams, cereal preparations, soluble coffee or cooked dishes.

11. Food supplement containing the concentrate according to one of Claims 1 to 5 in the form of pills, capsules or tablets containing a dose of 0.0001 to 100% of said concentrate.

## Patentansprüche

1. Natürliches Lycopenkonzentrat, extrahiert aus Tomatenpüree, Melone, Wassermelone, Guave, Pampelmuse, Aprikose und/oder aus Hagebutte, **dadurch gekennzeichnet, dass**
A - es bei Umgebungstemperatur wasserlöslich ist
B - es zumindest 1 mg Lycopen pro g des besagten Konzentrats, bis zu 30 % an Proteinen, bis zu 30 % an Polysacchariden, bis zu 10 % an organischen Säuren und zumindest 30 % an lipidischen Verbindungen enthält.

2. Konzentrat nach Anspruch 1, **dadurch gekennzeichnet, dass** es in pulvriger Form, flüssig oder geliert vorliegt.

3. Konzentrat nach Anspruch 1, **dadurch gekennzeichnet, dass** es ferner Vitamin E und/oder Vitamin C enthalten kann.

4. Konzentrat nach Anspruch 1, **dadurch gekennzeichnet, dass** es zwischen 1 mg und 40 mg Lycopen pro g an Konzentrat enthält.

5. Konzentrat nach Anspruch 4, **dadurch gekennzeichnet, dass** es vorzugsweise zwischen 10 und 30 mg an Lycopen pro g an Konzentrat enthält.

6. Verfahren zur Herstellung des Konzentrats nach einem der Ansprüche 1 - 5, wobei:
- man eine pflanzliche Struktur, die eine lipophile Zusammensetzung enthält, ausgewählt aus Tomatenpüree, Melone, Wassermelone, Guave, Pampelmuse, Aprikose und Hagebutte in wässriger Lösung alkalisiert,
- man die alkalisierte Lösung bis zum Sieden erhitzt, das man über 1 - 60 Minuten hält,
- man die Fasern und verschiedene unlösliche Komponenten durch Fest-Flüssig-Trennung isoliert,
- man in wässriger Lösung den erhaltenen Komplex ansäuert,
- man die Kohlenhydrate und andere unlöslichen Komponenten durch Fest-Flüssig-Trennung isoliert.

7. Verfahren nach Anspruch 6, **dadurch gekennzeichnet, dass** man dem Komplex Kalzium zufügt, um ein Gel zu erhalten.

8. Verfahren nach Anspruch 6, **dadurch gekennzeichnet, dass** man die Emulsion durch Zerstäuben oder Gefriertrocknung trocknet, um ein Pulver zu erhalten.

9. Topische kosmetische Zusammensetzung, die das Konzentrat nach einem der Ansprüche 1 - 5 enthält, zur Verlangsamung der Alterung der Haut und/oder zur Bekämpfung von Abbauvorgängen der Haut, die durch UV -Aussetzen der Haut erhalten werden können, wobei die Zusammensetzung zumindest 10⁻¹⁰ % Lycopen enthält.

10. Zusammensetzung, die auf oralem Weg einnehmbar ist, und die das Konzentrat nach einem der Ansprüche 1 - 5 enthält, um die Absorption von Lycopen zum Hervorrufen eines Lichtschutzes zu optimieren und um auch die Alterung der Haut zu verlangsamen, beispielsweise in Getränken, Schokolade, Eis oder Eiskrem, Getreidezbereitungen, löslichem Kaffee oder auch in Speisen.

11. Lebensmittelergänzung, die das Konzentrat nach einem der Ansprüche 1 - 5 in Form von Pillen, Gelkapseln oder von Tabletten in Dosen von 0,0001 bis 100 % des besagten Konzentrats enthält.
